# EUROPEAN PATENT APPLICATION

(11) **EP 1 630 555 A2**
(43) Date of publication of application: **01.03.2006**
(21) Application number: 04031064.1
(22) Date of filing: 30.12.2004
(51) Int. Cl.: G01N 33/487, G01N 35/10

(54) **High-throughput cell assay apparatus**

(30) Priority: 26.08.2004 JP 2004246150
(71) Applicant: Hitachi, Ltd., Tokyo (JP)
(72) Inventor: Asano, Yukako, Chiyoda-ku Tokyo (JP); Miyamoto, Tetsuro, Chiyoda-ku Tokyo (JP); Togashi, Shigenori, Chiyoda-ku Tokyo (JP)
(74) Representative: Beetz & Partner

(57) **Abstract**

In a high-throughput cell assay apparatus, a cell-and-electrode chip conveying apparatus (105) moves from a cell storage (101) to a measuring portion (102), and then to a cell-and-electrode chip disposal portion (108). Thereafter, it is determined if the electrode chip after being used should be re-used, again, or not, by referring to a condition for exchanging the electrode chip. When not be re-used, again, the cell-and-electrode chip conveying apparatus shifts the electrode chip from the measuring portion to the cell-and-electrode chip disposal portion, thereby disposing the electrode chip. The conveying apparatus moves from an exchangeable electrode chip storage (109) to the measuring portion, thereby position a new electrode chip onto the measuring portion.

## Description

The present application claims priority from Japanese application JP2004-246150 filed on August 26, 2004, the content of which is hereby incorporated by reference into this application.

### BACKGROUND OF THE INVENTION

The present invention relates to an assay apparatus for measuring bonding between a chemical compound and a receptor through the potential of membrane of a cell.

Because of an abrupt increase in recent years of chemical compounds, as being candidates for new medicines, it is strongly desired that an evaluation be conducted upon toxicity about large numbers of kinds of chemical compounds, through applying a method of the cell assay in which the measurement is obtained through measuring a membrane potential of a cell. As background, there is a potential difference of about several 10 mV (i.e., a static membrane potential) between an inside and an outside of the cellular membrane of a living cell; however, when letting an arbitrary receptor manifest upon the surface of cells, and when a chemical compound added thereto binds on the receptor, then an ion channel existing within the membrane opens so as to change an ion concentration between an inside and outside of the membrane. Then, current flows trough the membrane, thereby causing a change in the membrane potential.

With such method of cell assay obtainable through the measurement of membrane potential, it is possible to achieve the quantification of proteins easily from a current changing measurement with respect to a fixed membrane potential, and is also possible to obtain electrophysiological information directly and with high accuracy thereof, such as, the ion channel and/or transporter, etc. Results of those analyses can serve not only as contribution to designing of a method for a clinic test, but also as an effective means for selecting a chemical compound which can avoid risks upon a human being at an early-stage. For identifying chemical compounds of such large numbers, quickly, a high-throughput screening technology is necessary and indispensable.

Heretofore, regarding automation of such an apparatus for measuring the membrane potential, for example, in Japanese Patent Laying-Open No. 2001-201481 (2001), there is disclosed an electrophysiological automatic measuring apparatus in which an glass electrode is automatically inserted into the membrane of an oocyte to keep the membrane potential, thereby measuring reaction, automatically, when a medicine is dosed thereon. Also, Japanese Patent Laying-Open No. 2001-201481 (2001) discloses a method and a mechanism for selecting oocytes having a certain size through use of a filter, etc. , and separating one (s) having a certain membrane potential by measuring the membrane potential of the cells selected. Further, Japanese Patent Laying-Open No. Hei 9-5295 (1997) discloses a measuring apparatus applying a two (2) dimensional sensor for use in measuring cell activities, such as nerve cells, while making the position of a measuring electrode (or a probe) and/or the sizes thereof variable. And, Japanese Patent Laying-Open No. 2001-281201 (2001) discloses a unified or combined composite electrode having a plural numbers of fine or minute electrodes and wiring portions thereof, for recording electrical activities of nerve cells and so on, but within an outside of the cells.

In the future, it can be predicted that the requirement will increase for such the toxicity evaluation of chemical compounds, which is obtained through a cell assay method. In particular, there may be demand for an assay apparatus, which can evaluate toxicity automatically, upon a large variety of chemical compounds, as well as in the number of kinds thereof. However, with the cell assay system according to disadvantageous arrangement, the limited automation is achieved on the apparatus for measuring the membrane potential; however no consideration is paid upon a mechanism for exchanging the glass electrode easily and for disposal thereof. Accordingly, such fails to achieve a high-throughput system, which enables to evaluate a variety of chemical compounds being large in amount, as well as in the number of kinds thereof.

Also, a typical cell to be a target of evaluation may be very small, such as, about 1 mm in diameter for an oocyte, and from 10 µm to 20 µm in diameter for a zooblast or animal cell, for example. Such small size necessitates the craftsman-like skill of using a micro-manipulator or the like, to position the electrode and/or to insert the electrode into the cell, even if using an automatic apparatus. Therefore, such becomes an obstacle for achieving a high-throughput system which enables to evaluate a variety of chemical compounds being large in amount, as well as in the number of kinds thereof.

### BRIEF SUMMARY OF THE INVENTION

An object according to the present invention, is to provide a high-throughput cell assay apparatus which enables to evaluate a variety of chemical compounds being large in amount, as well as in the number of kinds thereof, with high accuracy.

According to the present invention, for achieving the object mentioned above, there is provided (1) a high-throughput cell assay apparatus for assaying bonding between a chemical compound and a receptor upon a basis of a value of an electric signal measured by electrodes contacting on a cell, which manifests an arbitrary receptor on a surface thereof and is added with a chemical compound thereto, comprising: an electrode chip being unified with an area for storing cells therein and electrodes to be contacted with the cell stored within said cell storing area; a measuring portion for measuring electric signals measured through said electrodes; and a cell and electrode conveying means being detachable with said electrode chip, and for connecting said electrodes thereof to said measuring portion, wherein said electrode chip attached on said conveying means can be exchanged with a new electrode chip.
(2) Also, according to the present invention, in item (1) mentioned above, preferably a condition for exchanging the electrode chip is determined depending upon a kind of cell to be measured. (3) Also, in item (1) mentioned above, preferably said cell and electrode conveying means comprises a driving mechanism for inserting the electrodes within the electrode chip into the cell.
(4) Also, preferably, item (1) mentioned above may further comprise a calculation processing portion for determining an electric signal obtained from a normal cell, from among electric signals obtained from plural numbers of cells onto which a same chemical compound is added at same time, thereby conducting statistic processing upon those electric signals.
(5) Also, in item (1) mentioned above, preferably said cell is made adhere onto a carrier, and the carrier on which the cell adheres, is stored within a cell storing area of said electrode chip by using said conveying means. (6) Also, in item (1) mentioned above, preferably, said cell is made to adhere onto a carrier, and a detachable combining mechanism is provided for conveying the carrier on which said cell adheres, or between the carrier on which said cell adheres at a specific position, and said electrode chip.
(7) Also, preferably, item (1) through (6) mentioned above may further comprise a cell storage enabling to store said cells in plural numbers thereof, a measuring portion for measuring the cells at positions thereof, an exchangable electrode chip storage for positioning plural numbers of said electrode chips therein, a cell and electrode chip disposal portion, and a transfer mechanism for transferring said cell and electrode chip conveying means to said cell storage, said measuring portion, said exchangable electrode chip storage and said cell and electrode chip disposal portion. (8) Also, in item (7) mentioned above, preferably, said cell and electrode conveying means comprises a means for picking up plural number of cells from said cell storage, a means for moving said cells picked up into an inside of said electrode chip, and a means for taking out said cells moved into the inside of said electrode chip, so as to dispose them into said cell and electrode chip disposal portion.

### BRIEF DESCRIPTION OF THE DRAWINGS

Those and other objects, features and advantages of the present invention will become more readily apparent from the following detailed description when taken in conjunction with the accompanying drawings wherein:
Fig. 1 is a vertical cross-section view showing a high-throughput cell assay apparatus according to an example embodiment of the present invention;
Fig. 2 is a perspective view of the high-throughput cell assay apparatus according to the example embodiment of the present invention;
Fig. 3 is a view for showing example carrier polystyrene beads, on which sample cells adhere;
Figs. 4A, 4B, 5-7, 8A and 8B show details of the high-throughput cell assay apparatus according to example embodiment of the present invention, wherein Fig. 4A is a front view of an example cell and electrode chip conveying apparatus, Fig. 4B shows an example electrode loading arrangement, Fig. 5 a block diagram of an example electrode chip, Fig. 6 a block diagram for showing an example relationship of electrical connection between a fine electrode and an outside, Fig. 7 an example operation flowchart, and Figs. 8A, 8B show example views explaining data procession; and
Figs. 9 to 11 are views for showing a high-throughput cell assay apparatus according to another example embodiment of the present invention, wherein Fig. 9 shows a vertical cross-section thereof, Fig. 10 a view for explaining a combination mechanism of a Mylar sheet and an electrode, and Fig. 11 a detailed cross-section view of an example lower portion of the electrode chip conveying apparatus.

### DETAILED DESCRIPTION

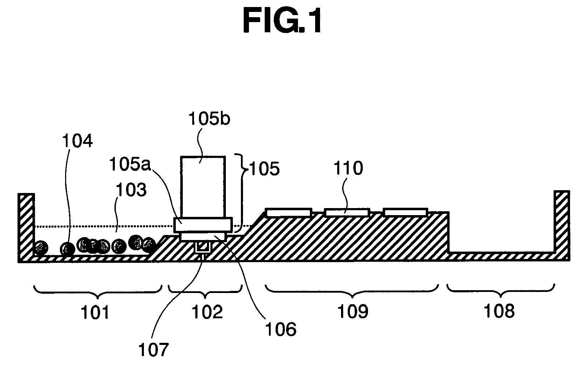

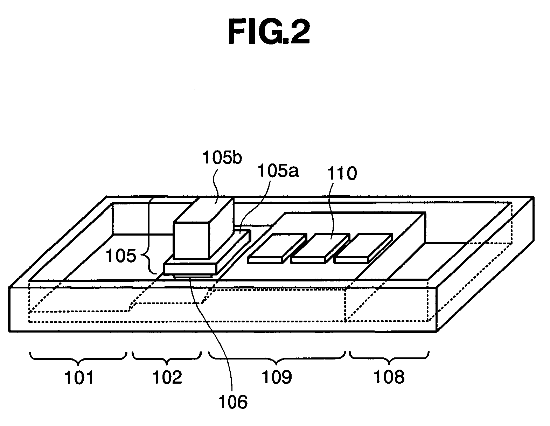

Hereinafter, example embodiments according to the present invention will be fully explained by referring to the attached drawings. Fig. 1 is a cross-section view for showing a brief structure of an example high-throughput cell assay apparatus, according to an embodiment of the present invention, and Fig. 2 is a perspective view of the high-throughput cell assay apparatus shown in Fig. 1. In the embodiment shown below, example explanation will be made about an embodiment of the present invention which accomplishes automation in relation to a handling method of cells and an exchange and disposal mechanism of an electrode. In particular, the taking up of HEK293 cells originated from a human kidney cell, such being adhesive animal cells from 10 µm to 20 µm in diameter thereof, will be used as an example. However, the high-throughput cell assay apparatus according to the present invention is not restricted only to the assay of such HEK293 cells, but it can be applied also to other types of cells.

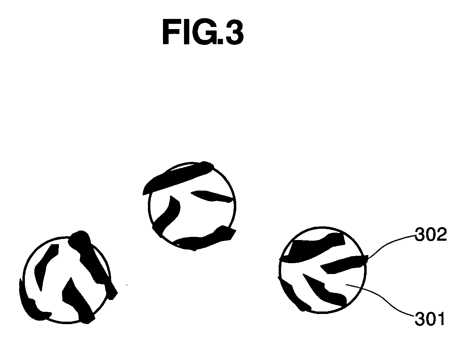

Fig. 3 is a view for showing example carrier polystyrene beads 301, on which the HEK293 cells 302 may adhere. When cultivating the HEK293 cells 302, the HEK293 cells 302 may stick on the surface of the beads 301 on which collagen coating has been made, for example, and are cultivated. Through cultivation of this manner, as is shown in Fig. 3, group of HEK293 cells 302 may grow up to be larger than a single body thereof, thereby improving a handling of the cells.

In the first embodiment of the present invention, example spherical or ball-like polystyrene beads 301 are used as a carrier, on the surface of which collagen coating is made, as shown in Fig. 3. However, the shape of the beads should not be necessarily limited to the spherical shape. As other non-limiting examples, they may be in a shape of a column, a cube, a rectangular parallelepiped, etc. , as long as the HEK293 cells 302 can adhere thereon. Further, in the present example embodiment, a reason of treatment of the collagen coating thereon is to improve an efficiency of adhesion of the HEK293 cells to the carrier. Such treatment of collagen coating is not always necessary, and/or the coating may be treated with alternative or other chemical materials. As is shown in Fig. 3, the HEK293 cells 302, adhering on the polystyrene beads 301 treated with the collagen coating, grow up while adhering around the polystyrene beads 301.

The example high-throughput cell assay apparatus shown in Fig. 1 may include a cell storage area 101, a measuring portion area 102, a culture medium 103, a cell-and-electrode chip conveying apparatus 105 (having a lower portion 105a and an upper portion 105b), an electrode chip 106, a cell-and-carrier suction opening 107, a cell-and-electrode chip disposal portion area 108, a spare or exchangeable electrode chip storage area 109, and also spare or exchangeable electrode chips 110. Further, in the cell storage area 101 are stored plural numbers of the polystyrene beads 104, on the surface of which the HEK293 cells adhere, as shown in Fig. 3. In the example shown in the Fig. , there is shown a case where three (3) pieces of the exchangeable electrode chips 110 can be stored within the exchangeable electrode chip storage area 109. However the number of the exchangeable electrode chips 110 is not limited necessarily only to three (3), but instead may be one (1), two (2) or four (4) or more. Also, in this example, the cell storage area 101, the measuring portion area 102, the exchangeable electrode chip storage area 109 and the cell-and-electrode chip disposal portion area 108 are disposed or aligned on a straight line as an example. However they are not limited to being aligned along the straight line, but they may be disposed, for example, in a radial or any other manner, in the place thereof.

As is shown in Fig. 1, the cell storage area 101 and the measuring portion area 102, are both made of stainless steel, and are filled with cultivating media 103. The polystyrene beads 104 (on the surface of which the HEK293 cells adhere) are prepared in plural numbers thereof, in advance, and disposed within the cell storage area 101. For conveying the polystyrene beads 104, from the cell storage area 101 to the measuring portion area 102, there may be used the cell-and-electrode chip conveying apparatus 105.

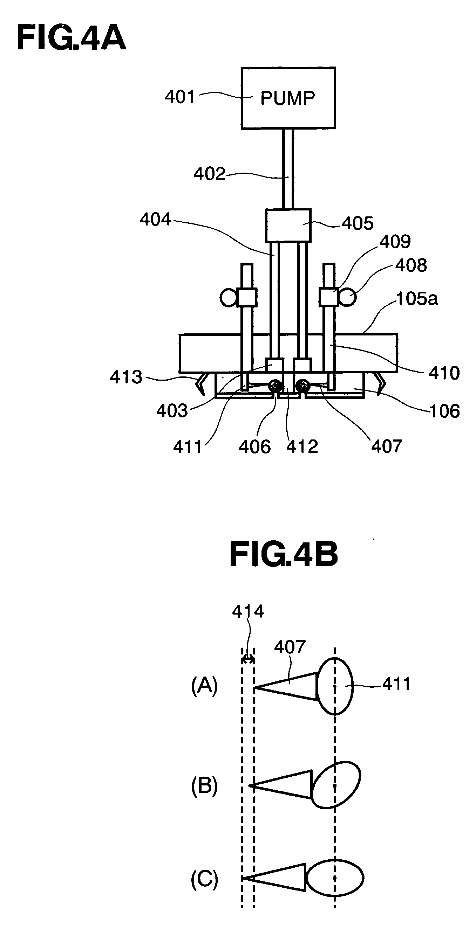

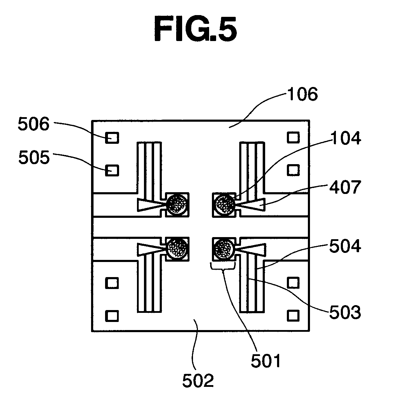

Figs. 4A and 4B show example structures of the cell-and-electrode chip conveying apparatus 105 within the high-throughput cell assay apparatus shown in Fig. 1 (having an example electrode chip 406 shown therein), wherein Fig. 4A is a block diagram of the structure, and Fig. 4B is a view for showing a cam mechanism for inserting a needle 407 (which is built in the fine or minute electrode (i.e., a fine electrode built-in needle)) into the HEK 293 cell. Also, Fig. 5 is a block diagram of the electrode chip 106 within the high-throughput cell assay apparatus according to the first embodiment. Further, in the example shown in the figure, there is shown the electrode chip 106, on which an example four (4) pieces of cell storage areas 501 are formed. However the number of pieces of the cell storage areas 501 is not limited to four (4), but it may be a number less than four (4) or exceeding that.

At appropriate times during operation, the cell-and-electrode chip conveying apparatus 105 is moved to the cell storage area 101, by means of an automatic conveying mechanism (not shown in the figure) or manually. And, as shown in Fig. 4A, polystyrene beads 104 having HEK293 cells stuck thereon are sucked into a cell-and-carrier hold portion 403, which is formed in a lower portion 105a of the cell-and-electrode chip conveying apparatus 105, due to suction developed by a pump 401 through a tube 402. Namely, as shown in Fig. 4A, upon a lower surface of the lower portion 105a of cell-and-electrode chip conveying apparatus 105 are formed plural numbers of the cell-and-carrier holding portions 403. And, tube 404 may be connected to each of those cell-and-carrier holding portions 403, respectively, through the lower portion 105a. Accordingly, due to the suction operation of the pump 401, the polystyrene bead 104 having the HEK293 cells stuck thereon is sucked into the cell-and-carrier holding portion 403.

In the example shown in the figure, the cell-and-carrier holding portions 403 are formed in four (4) pieces thereof, and four (4) flow passages by means of four (4) pieces of the tubes 404 are connected to the pump 401 through one (1) piece of the tube 402, by means of a joint 405. However, as one alternative, each of those four (4) pieces of the tubes 404 may be directly connected to the pump 401, respectively. Also, they may be connected to plural numbers of motors, while connecting the four (4) pieces of flow passages obtained by the four (4) pieces of the tubes 404, by means of the joints 405. While there is shown an example case where the cell-and-carrier holding portions 403 are four (4) in the number thereof, the number is not always limited to four (4). Further, although the pump 401 is applied into the method for sucking the cells and the carriers, however it may be possible to apply differing methods such as to apply water pressure, or the tube 404 can be sucked directly by a mouth. Also, it may be possible to provide a wastewater sink at a tip of the pump 401, thereby to collect the cultivating media, which was sucked out together with the cells and the carriers. The pump 401 may be applied a simple hand pump or a simple pump of electric motive type.

Following to the above, the cell-and-electrode chip conveying apparatus 105, having sucked therein the polystyrene bead 104 having the HEK293 cells stuck thereon, is conveyed from the cell storage area 101 to the measuring portion area 102, through a moving mechanism (e.g., toothed-rack, toothed gear and motor) or manually. And, when the cell-and-electrode chip conveying apparatus 105 reaches to a position on the electrode chip 106, the polystyrene beads 104 are discharged from the cell-and-carrier holding portions 403 due to a discharge operation of the pump 401, thereby to be stored within the cell storage areas 501 of the electrode chip 106 (shown in Fig. 5).

Upon a bottom surface of cell storage areas 501 of the electrode chip 106 is formed a cell-and-carrier suction hole 406 having such a size that the polystyrene bead 104 cannot slip out of the electrode chip 106, to fall down. In a lower portion of the measuring portion 102 is formed the cell-and-carrier suction opening 107; therefore, it is possible to charge the polystyrene bead 104 having the HEK293 cells stuck thereon into the cell storage areas 501 of the electrode chip 106, through sucking applied to that cell-and-carrier suction opening 107 by means of the pump (not shown in the figure). Further, in the example shown in the figure, four (4) cell-and-carrier suction holes 406 are formed, and are constructed so that four (4) flow passages connecting to those four (4) cell-and-carrier suctionholes 406 are communicating to a one (1) cell-and-carrier suction opening 107. Required is suction capacity for charging the polystyrene bead 104, on which the HEK293 cells adhere thereon, into cell storage areas 501 of the electrode chip 106. Alternatively to the above arrangement, it is also possible to use a one (1) flow passage covering the four (4) cell-and-carrier suction holes 406, or four (4) flow passages each connecting to the four (4) cell-and-carrier suction holes 406, respectively.

Also, in this example, though the pump is applied for sucking the cells and the carriers into the cell storage areas 501, it is also possible to apply other methods such as water pressure, for example, or the tube 404 can be sucked directly by a mouth of an operator. Further, it is also possible to provide a wastewater sink on the discharge side of the pump for sucking into the cell storage areas 501, thereby to collect cultivating media which was sucked out together with the cells and the carriers. Also, as shown in Fig. 1, there may be a difference in the height between the cell storage area 101 and the measuring portion area 102. Through keeping a liquid surface of the cultivation media 103 to be sufficiently high, it is possible to convey the polystyrene bead 104, on which the HEK293 cells adhere thereon, from the cell storage area 101 to the measuring portion area 102, while holding those within the cultivating media 103. As the method for moving the cell-and-electrode chip conveying apparatus 105, it is possible to apply an electric motor, or by using pressure obtained from an air, or it is also possible to move it on a rail by operating a handle, by directly operating it by hand. Such are non-limiting, non-exhaustive examples.

The electrode chip 106 may be manufactured through a technology of micromachining to result in an example form, as is shown in Fig. 5, mainly using a substrate 502 obtained by connecting silicon-silicon directly. The cell storage areas 501 and the fine electrode built-in needle 407 may be made up in one body; therefore, it is unnecessary to make a positioning between the cell and the fine electrode. The fine electrode built-in needle 407 may have two (2) fine electrodes 503 and 504, which are manufactured for use in an assay on the animal cell, and therefore it has such a structure that those two (2) fine electrodes 503 and 504 can be also inserted therein at the same time, when inserting a tip of the fine electrode built-in needle 407 into an inside of the cell.

As is shown in Fig. 4A, in order to insert the tip of the fine electrode built-in needle 407 into the HEK293 cell 302 on the polystyrene bead 104, first a toothed rotor 408 may be moved front and back (i.e., in a direction from the surface side to the reverse surface side of the paper), thereby bringing it to be meshed with a gear 409. Next, the gear 409 and a pushrod 410 are rotated around the longitudinal axes thereof. As a mechanism for moving the toothed rotor 408, there can be applied an electric motor, or it is also possible to apply the pressure obtained from an air, or it may be structured to move by hands, directly.

The electrode chip 106 located below the pushrod 410 may have a cam mechanism, as is shown in Fig. 4B; therefore, through rotation of the pushrod 410, a cam 411 rotates around, and then the fine electrode built-in needle 407 is pushed by the cam 411, so that it moves by a distance 414, as shown by example illustrations (A) → (B) → (C) in Fig. 4B. With this, the fine electrode built-in needle 407 is inserted into the HEK293 cell 302, while the polystyrene bead 104 on which the HEK293 cells adhere, is pushed onto a partition wall 412 defining part of the cell storage areas 501.

Although the cam 411, in the example shown in the figure, has an oval shape, it is not necessarily limited to such the oval shape; but it is sufficient to have such a shape, that the fine electrode built-in needle 407 moves through rotation of the cam 411, thereby to be inserted into the HEK293 cell 302 on the surface of polystyrene bead 104 sticking the HEK293 cells thereon. Building up the plural numbers of the cell storage areas 501 and the fine electrode built-in needles 407 into one (1) piece of silicon-silicon connecting substrate 502 enables such arrangement to insert the fine electrode built-in needles 407 into the plural numbers of the HEK293 cells 302, simultaneously or sequentially, thereby to measure the potential and current on those HEK293 cells 302.

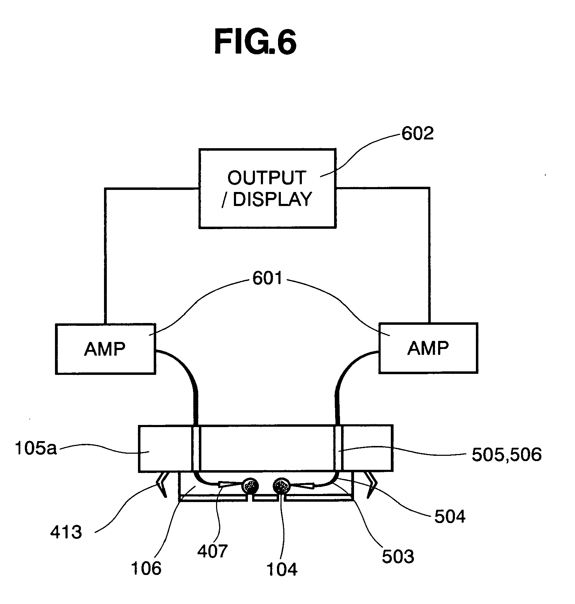

Fig. 6 is a block diagram for showing a relationship of electrical connection between the fine electrodes 503 and 504 and an outside in the cell-and-electrode chip conveying apparatus 105. In this Fig. 6, the fine electrodes 503 and 504 are connected to an amplifier 601 and an output recording and result displaying apparatus 602 through openings 505 and 506 for taking out the fine electrodes therethrough. Although in this example, one (1) fine electrode built-in needle 407 is connected to one (1) amplifier 601, it can also be constructed that plural numbers of fine electrode built-in needles 407 are connected to the amplifier 601, one by one, by exchanging the connection between the amplifier 601 and the fine electrodes 503 and 504 over time, electrically or mechanically.

After completing the measurement of the potential and current upon the cell, with sucking by using the pump 401 through the tube 402, the polystyrene beads 104 (still having the HEK293 cells stuck thereon) are sucked into the cell-and-carrier hold portions 403 of the cell-and-electrode chip conveying apparatus 105. Following thereto, the cell-and-electrode chip conveying apparatus 105 is conveyed to the cell-and-electrode chip disposal portion area 108. When the cell-and-electrode chip conveying apparatus 105 reaches up to the position of the cell-and-electrode chip disposal portion area 108, then the pump 401 starts a discharging operation, and the polystyrene beads 104 are completed, are disposed into the cell-and-electrode chip disposal portion area 108.

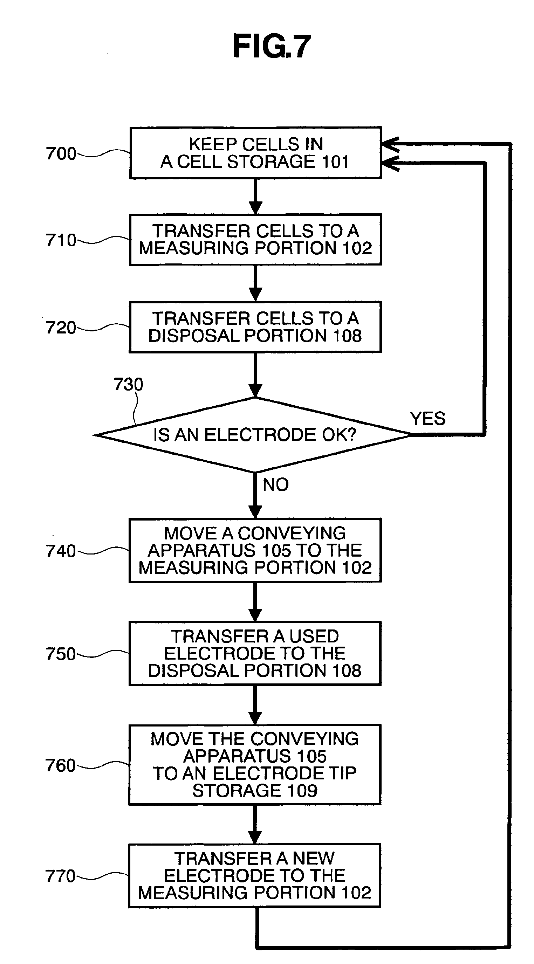

Fig. 7 shows an example operation flowchart of the example high-throughput cell assay apparatus shown in Fig. 1. In Fig. 7, there is shown an initial series of flows for conducting the cell assay operation, wherein the cell-and-electrode chip conveying apparatus 105 moves over time from the cell storage area 101 (operation 700) → the measuring portion area 102 (operation 710) → the cell-and-electrode chip disposal portion area 108 (operation 720). Thereafter, an operation controller portion (not shown in the figure) determines (operation 730) if the electrode chip should be re-used again or not, by referring to a condition for exchanging the electrode chips. In a case ("YES" branch) where it is decided to reuse the electrode, flow returns to operation 700.

In a case ("NO" branch) where it is decided not to re-use the spent electrode chip, a series of flows for exchanging and disposing the spent electrode chip is conducted. More particularly, the cell-and-electrode chip conveying apparatus 105 moves (operation 740) to the measuring portion area 102, so as to lift up the electrode chip 106, and moves (operation 750) to the cell-and-electrode chip disposal portion area 108, thereby disposing of the electrode chip 106. Thereafter, the cell-and-electrode chip conveying apparatus 105 moves (operation 760) to the exchangeable electrode chip storage area 109, so as to lift up a new electrode chip 106, and moves (operation 770) to the measuring portion area 102, thereby positioning the new electrode chip 106 on the measuring portion 102. The above decision and operation may be conducted automatically (e.g., without requirement of human intervention) through suitable programming, such programming being well within a purview of one skilled in the art.

Herein, as one example condition, for exchanging, it may be possible to determine exchange over time when the electrode chip 106 is re-used five (5) times, for example, by taking the lifetime of the electrode chip 106 into the consideration. However, the number of times for re-using is not necessarily limited to five (5) times, since a recommended number of reuses depends upon the materials and the performances of the electrode chip 106. In order to exchange after being used five (5) times, the spent electrode chip 106 is lifted up at a central portion among the four (4) sides thereof by means of hooks 413, which are formed on the lower portion of the cell-and-electrode chip conveying apparatus 105, and then the spent electrode chip 106 is removed from the measuring portion area 102. And, when the cell-and-electrode chip conveying apparatus 105 retracts the hooks 413 therefrom, then the spent electrode chip 106 is disposed into the cell-and-electrode chip disposal portion area 108. Lifting up of a new electrode chip 106 is also conducted by using the hooks 413 in a similar manner, and the same for disposing it onto the measuring portion area 102.

Herein, a driving means of the hooks 413 may be an electric means (such as, an electric motor), may be magnetism (such as, a permanent magnet), or may be a manual mechanism. However, in this example, though it is explained that the electrode chip 106 is conveyed and disposed by means of the hooks 413 which are formed in the lower portion of the cell-and-electrode chip conveying apparatus 105, practice of the invention is not limited to using such hooks 413, as it is possible to convey and dispose the electrode chip 106 in a different way. For example, in the similar manner as conveying the polystyrene bead 104 sticking the HEK293 cells thereon, the electrode chip 106 can be also conveyed and disposed by sucking and separating the electrode chip 106 through the function of the pump.

Continuing discussion, since the normal HEK 293 cell 302 shows a static membrane potential from -70 mV to -10 mV in the value thereof, it is possible to confirm that the HEK 293 cell 302 is under a predetermined condition or not during suitable measurement. Then, using such information, it is possible to determine the condition for exchanging the electrode chip. This is because, the cases showing an abnormal value on the static membrane potential include not only the case where the HEK 293 cell 302 is under the condition not being suitable for measurement, but also the case where an abnormality is caused in the electrode chip 106, such as, breakage of the fine electrode built-in needle 407, breakage of the fine electrodes 503 and 504, and/or blockage of the flow passage due to damaged cells, etc.

As further discussion, the value of the static membrane potential of the HEK 293 cell 302 may be recorded into the output recording and result displaying apparatus 602 for each of the cell assays within the measuring portion 102. As an example condition for exchanging the electrode cell 106 after being used, for example, it may be when measurement cannot be made on half or more of the entire numbers of plural cell storage areas 501 within a chip, and if the fine electrode built-in needle 407 within a certain cell storage areas 501 is still able to measure or not, it can be also determined with the value of the static membrane potential of the HEK 293 cell 302 stored within that cell storage areas 501.

Further, in the example mentioned above, the condition for exchanging the electrode chip 106 after being used is made when measurement cannot be made on half or more of the entire numbers of the plural cell storage areas 501; however it is also possible to change a ratio of the number of the cell storage areas where the measurement cannot be made, to be the condition for exchanging the electrode chip 10 6 after being used. Such may depend, for example, upon the factors, such as, a number of the cell storage areas 501 included within one (1) piece of the electrode chip 106, or on the number of the cell storage areas 501 to be measured at one time, etc. For example, in a case when the value of the static membrane potential of the HEK 293 cell 302 stored within the same cell storage area 501 does not stay within the region from -70 mV to -10 mV continuously five (5) times, it may be determined that the measurement cannot be made within that cell storage area 501. However, the number of the times need not be limited to five (5) times, and any suitable number may be used as a reference.

Also, the region of values on the static membrane potential for the normal HEK 293 cell 302 may be determined from -70 mV to -10 mV to be the reference for determination, in the above. However for increasing the quality of the HEK 293 cell 302 as a target of measurement, it is also possible to vary (e.g., narrow) the region of the value of the reference for determination, such as, from -70 mV to -20 mV, for example. And, at a stage when the number of the cell storage areas 501 in which the measurement cannot be made on the electrode chip 106 comes down to be half of the entire number of the cell storage areas 501 or lower than that, that electrode chip 106 is determined disposed as being a spent electrode chip. As another example, a chemical compound may be added onto the plural numbers of normal HEK 293 cells 302, and a statistic process maybe applied upon a measurement result obtained therefrom, thereby enabling to improve accuracy on the measurement.

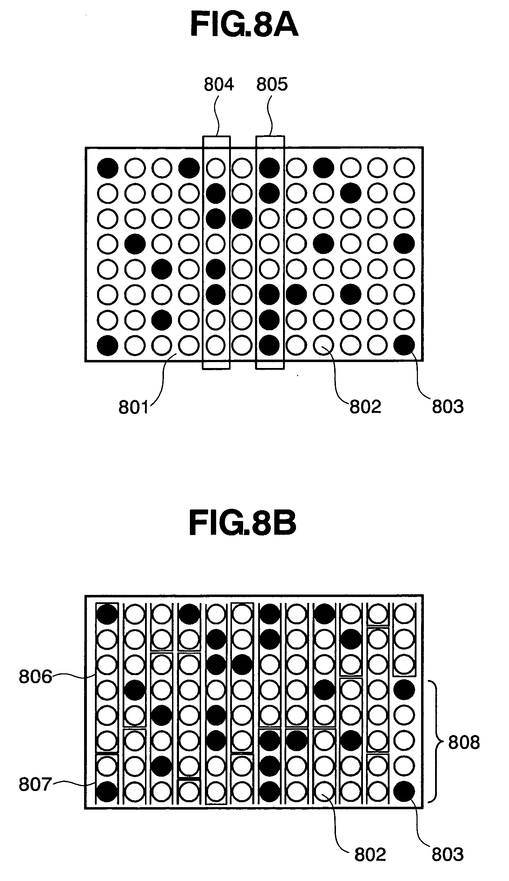

Figs. 8A and 8B are block diagrams for explaining an example of a data selection method of conducting an example statistic process, for the purpose of improving an accuracy of the measurement. As is shown in Fig. 8A, for example, consideration will be paid on an electrode chip 801, which includes 96 pieces of cell storage areas of in an array of 8 lines x 12 rows therein. For example, in a case of improving the accuracy on the measurement through an arithmetical mean of the measurement results obtained, the condition for re-using the electrode chip 801 again may be determined based upon if there are five (5) pieces of normal HEK 293 cells or not upon which the same chemical compound can be added at the same time. Herein, if the HEK 293 cells are normal or not, can be determined from the values of the static membrane potentials thereof, in the similar manner as was mentioned above. Though consideration is made that the arithmetical mean, is conducted upon the data obtained from the five (5) pieces of cells, in this example, the number of data to be conducted with the arithmetical mean is not necessarily limited to five (5) pieces. For example, a weighted means can be applied as a method for the statistic processing thereof.

As is shown in Fig. 8A, from the values of the static membrane potential on the cells, they are determined to be example cells 802 (a white circle) which indicate a normal value on the static membrane potential, or example cells 803 (a black circle) which indicate an abnormal value on the static membrane potential, and the position information thereof is memorized into the output recording and result displaying apparatus 602. For example, paying an attention onto each one of the vertical rows, among twelve (12) rows, the rows 804 and 805, having cells 802 indicating the normal value on the static membrane potential does not reach up to five (5), are determined and made unusable no addition of the chemical compound is made upon such rows.

In contrast, any row in which the number of cells 802 indicating the normal value on the static membrane potential is five (5) or more, is made usable, and addition of the chemical compound is conducted thereupon. And, at a stage when all rows are made unusable, the chip is disposed as a spent electrode chip. Herein, in the example shown in Fig. 8A, determination is made by paying attention onto the rows; however an alternative may be made by paying attention onto the lines in the place thereof. It is also possible to dispose of a chip as a spent electrode chip at a stage when several (e.g., a predetermined number) of the twelve (12) rows are made unusable. Further, in this example, the consideration is paid on the case where the arithmetical mean is made on the values obtained from the five (5) pieces of the HEK 293 cells 302; however, the number of the data on which the arithmetical mean is made, need not always be five (5).

Further, it may also be possible to freely select a combination of the HEK 293 cells 302, for example, onto which addition of the same chemical compound will be made so that each includes therein five (5) pieces of the cells 802, each of which indicates a normal value on the static membrane potential. Namely, as is shown in Fig. 8B, it is also possible to freely make selection so that five (5) pieces of the cells 802 indicating a normal value on the static membrane potential are included by each, sequentially, from a top of the most left-hand side row. Further, it is also possible to make selection so that the five (5) pieces are included by each, sequentially from the bottom of the most left-hand side row, or sequentially from the top of the most right-hand side row, and further, sequentially from the bottom of the most right-hand side row. Also, determination may be made, not paying an attention onto the row, but on the line in the place thereof.

Furthermore, while memorizing the cell storage areas 501 (each being attached with a number) into the output recording and result displaying apparatus 602, it is also possible to freely select a combination of the HEK 293 cells 302, onto which addition of the same chemical compound will be made, so that each includes five (5) pieces of cells 802. And then, to the fourteen (14) combinations of such the combinations 806, 807, etc., for example, each including therein five (5) pieces of the cells indicating the normal value on the static membrane potential, the same chemical compound is added at the same time, respectively, while addition is not made onto the cells 808 which are not applicable to the group. And at the stage when it is impossible to make up a combination including five (5) pieces of the cells 802 which indicate the normal value on the static membrane potential, then it may be disposed as being a spent electrode chip. Though the consideration is made that the arithmetical mean is conducted upon the data obtained from five (5) pieces of the HEK 293 cells 302 in this example, the number of the data upon which the arithmetical mean is conducted need not always be five (5).

Also, in this example, addition of the same chemical compound is made onto the cells 803 which indicate a normal value on the static membrane potential. However in the case where the chemical compound to be added is rare or it is expensive, it is also possible to made addition of the same chemical compound only onto fewer of the cells 802 indicating the normal value on the static membrane potential. Also, using the cell assay system mentioned above, but targeting the measurement upon an oocyte or an egg cell while changing the fine electrodes 503 and 504 to be used for the oocyte or the egg cell, enables to conduct a cell assay operation upon the oocyte or the egg cell. Further, the operation controller portion may be provided in alternative ways not shown in the figure, such as being provided within the cell-and-electrode chip conveying apparatus 105.

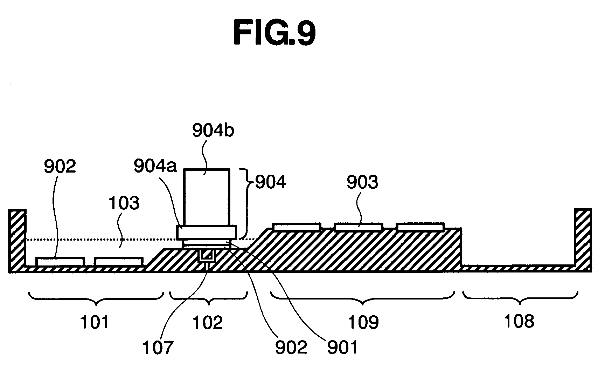

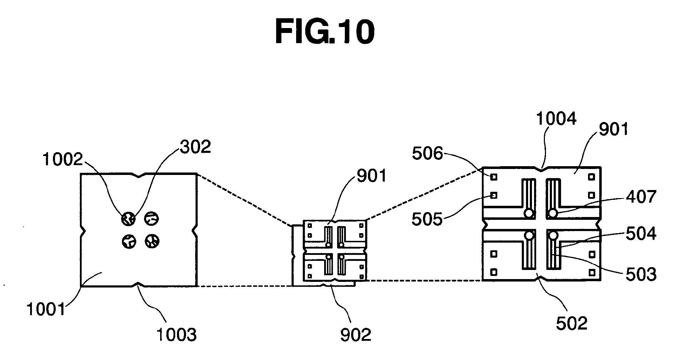

Fig. 9 is a cross-section view for showing a brief structure of a high-throughput cell assay apparatus according to a second embodiment of the present invention, while Fig. 10 is a view for showing a method for combining a Mylar sheet on which the HEK 293 cells adhere and the electrode chip, within the high-throughput cell assay apparatus shown in Fig. 9. In Figs. 9 to 11, there can be a cultivating of the HEK 293 cells on a Mylar sheet 1001 being the same size as the electrode chip. For example, when a thiol radial 1002 is introduced at the position corresponding to the tip of the fine electrode built-in needle 407, as a vehicle to cultivate the HEK 293 cells 302, then the HEK 293 cells 302 will selectively adhere at the position where the thiol radial 1002 is introduced.

Cultivation thereof in such a manner brings the HEK 293 cell to be large in appearance, comparing to that of the single body of the HEK 293 cell 302, as shown in Fig. 10, thereby enabling improved handling of the cells. Though there is used a Mylar sheet 1001, in this example, however practice of the invention is not limited to the Mylar sheet 1001, but it may be other types of sheet-like materials on which the HEK 293 cells 302 can adhere. Also, the thiol radial 1002 is introduced onto the Mylar sheet 1001 in the present embodiment, and this introduction of the thiol radial 1002 is for the purpose of promoting the adhesion of the HEK 293 cell 302 onto a specific position; however, practice of the present invention is not limited to the thiol radial 1002, but other types of chemical compound may be introduced, as long as it can promote the adhesion of the HEK 293 cell 302. Further, in the present embodiment, there is shown the Mylar sheet 1001, on which the thiol radial 1002 is introduced at four (4) places thereof ; however the number of the places the number is not limited only to the number four (4).

Between the Mylar sheet 902 on which the HEK 293 cells adhere and the electrode chip 901, there may be provided a combining or coupling mechanism allowing the sheet/chip to be detachable to each other. With provision of such mechanism, it may be possible to insert the fine electrode built-in needle 407 into the HEK 293 cell 302, with high efficiency. Regarding the needle management, on the electrode chip 901 there may be formed the fine electrodes 503 and 504 and the openings 505 and 506 for taking out the fine electrodes therethrough, as shown in Fig. 10.

Also, as is shown in Fig. 10, there may be formed cuttings (e.g., indexes) 1003 and 1004, in advance, at a center on the respective four (4) sides of the Mylar sheet 902, and the electrode chip 901; therefore, when combining the Mylar sheet 902 and the electrode chip 901, the positioning therebetween may be obtained easily, through fitting or registering those cuttings 1003 and 1004 with respect to one another.

In the present embodiment, the cuttings may be formed in the shape of a triangle at the center on the respective four (4) sides of the Mylar sheet 902, and the electrode chip 901; however, the shape thereof is not limited to a triangle, and instead cuttings formed in a semicircle or rectangular shape may be adopted, e.g., as long as they can achieve the positioning therebetween. Also, the position where each cutting is formed is not limited to the center on the respective four (4) sides of the Mylar sheet 902, and the electrode chip 901; they may be formed at non-center portions , or differing combinations of positions.

As is shown in Fig. 9, the Mylar sheet 902 on which the HEK 293 cells adhere, is prepared (e.g., cultured) within the cell storage area 101, in advance. Then, using an electrode chip conveying apparatus 904, the Mylar sheet 902 on which the HEK 293 cells adhere, may be taken out from the cell storage area 101 by means of the hooks 413, and conveyed to the measuring portion area 102, to be positioned therein. Following thereto, a Mylar sheet-and-electrode chip conveying apparatus 904 (having lower portion 404A and upper portion 404B) may take out the electrode chip 901 from the exchangeable electrode chip storage area 109, to convey it to the measuring portion 102, and as shown in Fig. 10, the Mylar sheet 902, and the electrode chip 901 are combined together.

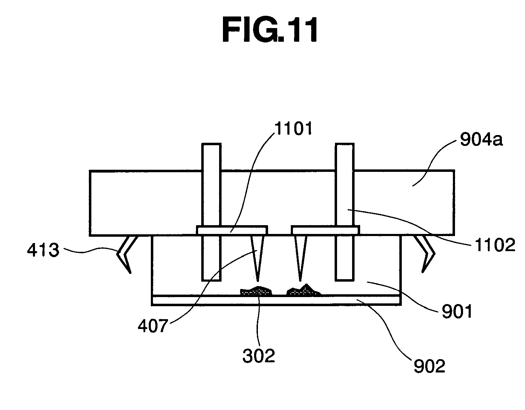

Fig. 11 is a cross-section view for showing an outlook of a lower portion 904a of the Mylar sheet-and-electrode chip conveying apparatus 904 and the Mylar sheet 902 and the electrode chip 901, within the high-throughput cell assay apparatus shown in Fig. 9. As is shown in Fig. 11, in order to insert a tip of the fine electrode built-in needle 407 of the electrode chip 901 into the HEK 293 cell 302 on the Mylar sheet 902, a supporting rod 1101 connected to a push-rod 1102 of the lower portion 904a is shifted downwards together with the push-rod 1102. With this, the fine electrode built-in needle 407 is pushed down by means of the supporting rod 1101, so as to move downwards, thereby inserting the needle into the HEK 293 cell 302. However, if moving the supporting rod 1101 is erroneously inserted too much, i.e., more than an amount necessary, the fine electrode built-in needle 407 may break through the HEK 293 cell 302 to reach the surface of the Mylar sheet 902; i.e., there is a danger that the fine electrode built-in needle 407 may be damaged. For this reason, it is preferable that the length of the push rod 1102 and the length of the supporting rod 1101 are limited in some way such that the fine electrode built-in needle 407 cannot break through the HEK 293 cell 302.

As a means for pushing the push rod 1102 downward, such may be accomplished by using an electric motor, magnetism, air pressure, or a manual means, etc. After completing the measurement in the measuring portion 102, the Mylar sheet and the Mylar sheet-and-electrode chip conveying apparatus 904 moves to the cell-and-electrode chip disposal portion 108, thereby disposing the Mylar sheet 902 on which the HEK 293 cells adhere.

Herein, in a case similar to that applied in the first embodiment of the present invention, the electrode chip 901 may also be disposed into the cell-and-electrode chip disposal portion 108. And, in the similar manner as was mentioned above, after moving a new Mylar sheet 902 on which the HEK 293 cells adhere from the cell storage area 101 into the measuring portion area 102, a new electrode chip 903 (i.e., an exchangeable electrode chip) may be taken out from the exchangeable electrode chip storage area 109.

However, in a case when not disposing of the electrode chip 901, the next Mylar sheet 902, being supplied from the cell storage area 101 is combined to the reused electrode chip 901, so as to make the measurement thereon in the measuring portion area 102. Namely, even in the cell assay apparatus shown in Fig. 9, it is possible to conduct the cell assay with the control algorithm that is similar to that shown in Fig. 7.

With the mechanism according to the example second embodiment, the electrode chip 901 is combined on top of the Mylar sheet 902 on which the HEK 293 cells adhere; however turning themupside-down, the Mylar sheet 902 on which the HEK 293 cells adhere may be combined on top of the electrode chip 901, thereby conducting the cell assay thereon. And, in the second embodiment, there also may be provided the amplifier 601 and the display apparatus 602, which are shown in Fig. 6 mentioned above, in the similar manner to that of the first embodiment.

As was fully explained in the above, according to the present invention, it is possible to achieve a high-throughput cell assay apparatus enabling automatic operations upon a large amount and large numbers of chemical compounds in a short time, but with high accuracy. Further, the automation of an exchanging and disposal mechanism for the electrode, and also the handling of the fine cells, enables one to obtain the high-throughput cell assay, thereby enabling identification upon large numbers of kinds of the chemical compounds, quickly.

Embodiments of the present invention may afford a high degree of automatic operations (i.e., not requiring human intervention). More particularly, an automatic arrangement (amotor, pump, sensors, etc. operating in conjunction with a suitably programmed processor) may automatically convey the chip, cell carrier(s) and/or sample cell(s) between ones of the cell storage portion, measuring portion, exchangeable electrode chip storage portion and cell/electrode chip disposal portion. At the cell storage portion, the automatic arrangement may automatically load cell carrier(s) and/or sample cell(s) into the chip, and at the cell/electrode chip disposal portion the automatic arrangement may automatically unload cell carrier(s) and/or sample cell(s) from the chip. At the measuring portion, the automatic arrangement may automatically apply chemicals onto the sample cell (s), and/or provide automated testing of the sample cell(s). Still further, the automatic arrangement may automatically perform chip testing/analysis to decide whether a chip is to be reused or replaced. Upon replacement determination, at the cell/electrode chip disposal portion, the automatic arrangement may automatically unload (i.e., discard) the chip, and at the exchangeable electrode chip storage portion, the automatic arrangement may automatically reload a replacement chip. Still further, the automatic arrangement may be programmed to alert/request human intervention upon predetermined occurrences (e.g., failures, lack of replacement chips within the exchangeable electrode chip storage portion). Design of components and programming to construct any portion or whole of the above automatic arrangement is well within a purview of persons skilled within the present art to which the present invention is directed.

The preferred embodiment described herein are therefore illustrative and not restrictive, the scope of the invention being indicated by the appended claims and all variations which comes within the meaning of the claims are intended to be embraced therein.

## Claims

1. A high-throughput cell assay apparatus, comprising:
an electrode chip (106) having at least one cell storing area (101) for storing at least one cell therein, and electrodes to be contacted with the cell stored within said cell storing area;
a measuring portion (102) to measure electric signals measured through said electrodes; and
a cell and electrode conveying means (105), being detachable with said electrode chip, and for connecting said electrodes thereof to said measuring portion, wherein said electrode chip attached on said conveying means can be automatically exchanged with a new electrode chip (110).

2. The high-throughput cell assay apparatus as described in claim 1, wherein a condition for exchanging the electrode chip is determined depending upon a kind of the cell to be measured.

3. The high-throughput cell assay apparatus as described in claim 1, wherein said conveying means comprises a driving mechanism for inserting the electrodes within said electrode chip into the cell.

4. The high-throughput cell assay apparatus as described in claim 1, comprising a calculation processing portion for determining an electric signal obtained from a normal cell from among electric signals obtained from plural numbers of cells, onto which a same chemical compound is added, thereby conducting statistic processing upon those electric signals.

5. The high-throughput cell assay apparatus as described in claim 1, wherein said cell is made adhere onto a carrier, and the carrier on which the cell adheres is stored within the cell storing area of said electrode chip by using said conveying means.

6. The high-throughput cell assay apparatus as described in claim 1, wherein said cell is made adhere onto a carrier, and a detachable combining mechanism is provided for conveying the carrier and said electrode chip.

7. The high-throughput cell assay apparatus as described in claim 1, comprising a cell storage portion to store said cell in plural numbers thereof, a measuring portion for measuring the cell at positions thereof, an exchangeable electrode chip storage portion (109) for positioning plural numbers of said electrode chips therein, a cell and electrode chip disposal portion (108), and a transfer mechanism to automatically transfer said conveying means between ones of said cell storage portion, said measuring portion, said exchangeable electrode chip storage portion and said cell and electrode chip disposal portion.

8. The high-throughput cell assay apparatus as described in claim 7, wherein said conveying means comprises a means for picking up plural number of cells from said cell storage, a means for moving said cells picked up into an inside of said electrode chip, and a means for taking out said cells moved into the inside of said electrode chip, so as to dispose them into said cell and electrode chip disposal portion.

9. The high-throughput cell assay apparatus as described in claim 3, comprising a cell storage portion to store said cell in plural numbers thereof, a measuring portion for measuring the cell at positions thereof, and exchangeable electrode chip storage portion (109) for positioning plural numbers of said electrode chips therein, a cell and electrode chip disposal portion (108), and a transfer mechanism to automatically transfer said conveying means between one of said cell storage portion, said measuring portion, said exchangeable electrode chip storage portion and said cell and electrode chip disposal portion.

10. The high-throughput cell assay apparatus as described in claim 9, wherein said conveying means comprises a means for picking up plural number of cells from said cell storage, a means for moving said cells picked up into an inside of said electrode chip, and a means for taking out said cells moved into the inside of said electrode chip, so as to dispose them into said cell and electrode chip disposal portion.

11. The high-throughput cell assay apparatus as described in claim 4, comprises a cell storage portion to store said cell in plural numbers thereof, a measuring portion for measuring the cell at positions thereof, an exchangeable electrode chip storage portion for positioning plural numbers of said electrode chips therein, a cell and electrode chip disposal portion, and a transfer mechanism to automatically transfer said conveying means between ones of said cell storage portion, said measuring portion, said exchangeable electrode chip storage portion and said cell and electrode chip disposal portion.

12. The high-throughput cell assay apparatus as described in claim 11, wherein said cell and electrode conveying means comprises a means for picking up plural number of cells from said cell storage, a means for moving said cells picked up into an inside of said electrode chip, and a means for taking out said cells moved into the inside of said electrode chip, so as to dispose them into said cell and electrode chip disposal portion.

13. The high-throughput cell assay apparatus as described in claim 6, comprising a cell storage portion to store said cell in plural numbers thereof, a measuring portion for measuring the cell at positions thereof, an exchangeable electrode chip storage portion for positioning plural numbers of said electrode chips therein, a cell-and-electrode chip disposal portion, and a transfer mechanism to automatically transfer said conveying means between ones of said cell storage portion, said measuring portion, said exchangeable electrode chip storage portion and said cell and electrode chip disposal portion.

14. The high-throughput cell assay apparatus as described in claim 13, wherein said cell and electrode conveying means comprises a means for picking up plural number of cells from said cell storage, a means for moving said cells picked up into an inside of said electrode chip, and a means for taking out said cells moved into the inside of said electrode chip, so as to dispose them into said cell and electrode chip disposal portion.

15. The high-throughput cell assay apparatus as described in claim 1, wherein the cell and electrode conveying means includes an automatic arrangement for automatically conveying the electrode chip between predetermined locations of the high-throughput cell assay apparatus.

16. The high-throughput cell assay apparatus as described in claim 1, wherein the cell and electrode conveying means includes an automatic arrangement for automatically loading/unloading cell carriers and/or cells into/from the electrode chip.

17. The high-throughput cell assay apparatus as described in claim 1, wherein the cell and electrode conveying means includes an automatic arrangement for automatically applying a chemical onto the cell, and/or automated testing of the cell.

18. The high-throughput cell assay apparatus as described in claim 1, wherein the cell and electrode conveying means includes an automatic arrangement for automatically performing chip testing/analysis to decide whether the electrode chip to be reused or replaced.

19. The high-throughput cell assay apparatus as described in claim 1, wherein the cell and electrode conveying means includes an automatic arrangement for automatically loading a presently-loaded electrode chip, and automatically loading a replacement electrode chip.
